(19) Europäisches Patentamt
European Patent Office
Office européen des brevets

(11) EP 1 868 591 B1

(12) **EUROPEAN PATENT SPECIFICATION**

(45) Date of publication and mention of the grant of the patent:
**30.05.2012 Bulletin 2012/22**

(21) Application number: **06748308.1**

(22) Date of filing: **07.03.2006**

(51) Int Cl.:
***A61B 19/00*** *(2006.01)* ***A61N 1/00*** *(2006.01)*
***A61N 2/00*** *(2006.01)* ***C12N 13/00*** *(2006.01)*
***C12M 1/00*** *(2006.01)* ***A61N 2/02*** *(2006.01)*

(86) International application number:
**PCT/US2006/008042**

(87) International publication number:
**WO 2006/096698 (14.09.2006 Gazette 2006/37)**

(54) **PHARMACOLOGICAL, CHEMICAL, AND TOPICAL AGENT ENHANCEMENT APPARATUS**

VORRICHTUNG ZUR VERSTÄRKUNG PHARMAKOLOGISCHER, CHEMISCHER UND TOPISCHER WIRKSTOFFE

APPAREIL SERVANT A RENFORCER L'EFFET D'UN AGENT PHARMACOLOGIQUE, CHIMIQUE ET TOPIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.03.2005 US 658968 P**
**06.03.2006 US 369309**

(43) Date of publication of application:
**26.12.2007 Bulletin 2007/52**

(73) Proprietor: **Ivivi Health Sciences, LLC**
**San Francisco, CA 94107 (US)**

(72) Inventors:
- **PILLA, Arthur, A.**
**Oakland, New Jersey 07436 (US)**
- **DIMINO, Andre, A.**
**Woodcliff Lake, New Jersey 07677 (US)**

(74) Representative: **Schlich, George William et al**
**Schlich LLP**
**34 New Road**
**Littlehampton, West Sussex BN17 5AT (GB)**

(56) References cited:
**WO-A2-2004/108208** **GB-A- 2 400 316**
**US-A1- 2001 044 643** **US-A1- 2002 035 358**
**US-A1- 2004 267 333** **US-A1- 2005 049 640**

- **LANSDOWN, A.B.G. ET AL.: 'Sequential Changes in Trace Metal, Metallothionein and Calmodulin Concentrations in Healing Skin Wounds.' J. ANAT. vol. 195, 1999, pages 375 - 386, XP008091371**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 868 591 B1

## Description

This invention relates to enhancing effectiveness of pharmacological, chemical, cosmetic and topical agents used to treat living tissues, cells and molecules by altering the interaction with the electromagnetic environment of the living tissues, cells, and molecules. More particularly, this invention provides for an application of highly specific electromagnetic frequency ("EMF") signal patterns to one or more body parts by surgically non-invasive reactive coupling of encoded electromagnetic information. Such application of electromagnetic waveforms in conjunction with pharmacological, chemical, cosmetic and topical agents as applied to, upon, or in human, animal, and plant target pathway structures such as cells, organs, tissues and molecules, can serve to enhance various effects of such agents.

## BACKGROUND ART

The use of most low frequency EMF has been in conjunction with applications of bone repair and healing. As such, EMF waveforms and current orthopedic clinical use of EMF waveforms comprise relatively low frequency components and are of a very low power, inducing maximum electrical fields in a millivolts per centimeter (mV/cm) range at frequencies under five KHz. A linear physicochemical approach employing an electrochemical model of cell membranes to predict a range of EMF waveform patterns for which bioeffects might be expected is based upon an assumption that cell membranes, and specifically ion binding at structures in or on cell membranes, are a likely EMF target. Therefore, it is necessary to determine a range of waveform parameters for which an induced electric field could couple electrochemically at a cellular surface, such as by employing voltage-dependent kinetics. Extension of this linear model involves Lorentz force considerations that eventually demonstrated that the magnetic component of EMF could play a significant role in EMF therapeutics. This led to the ion cyclotron resonance and quantum models that predicts benefits from combined AC and DC magnetic field effects at very low frequency ranges.

A pulsed radio frequency ("PRF") signal derived from a 27.12 MHz continuous sine wave used for deep tissue healing is known in the prior art of diathermy. A pulsed successor of the diathermy signal was originally reported as an electromagnetic field capable of eliciting a non-thermal biological effect in the treatment of infections. Subsequently, PRF therapeutic applications have been reported for the reduction of post-traumatic and post-operative pain and edema in soft tissues, wound healing, burn treatment, and nerve regeneration. The application of PRF for resolution of traumatic edema has become increasingly used in recent years. Results to date using PRF in animal and clinical studies suggest that edema may be measurably reduced from such electromagnetic stimulus.

The within invention is based upon biophysical and animal studies that attribute effectiveness of cell-to-cell communication on tissue structures' sensitivity to induced voltages and associated currents. A mathematical analysis using at least one of a Signal to Noise Ratio ("SNR") and a Power Signal to Noise Ratio ("Power SNR") evaluates whether EMF signals applied to target pathway structures such as cells, tissues, organs, and molecules, are detectable above thermal noise present at an ion binding location. Prior art of EMF dosimetry did not taken into account dielectric properties of tissue structures, rather the prior art utilized properties of isolated cells. By utilizing dielectric properties, reactive coupling of electromagnetic waveforms configured by optimizing SNR and Power SNR mathematical values evaluated at a target pathway structure can enhance various effects of pharmacological, chemical, cosmetic and topical agents that are applied to, upon or in human, animal and plant cells, organs, tissues and molecules. An enhancement results from increased blood flow and modulation of angiogenesis and neovascularization as well as from other enhanced bioeffective processes.

Recent clinical use of non-invasive PRF at radio frequencies has used pulsed bursts of a 27.12 MHz sinusoidal wave, each pulse burst typically exhibiting a width of sixty five microseconds and having approximately 1,700 sinusoidal cycles per burst, and with various burst repetition rates.

Broad spectral density bursts of electromagnetic waveforms having a frequency in the range of one to one hundred megahertz (MHz), with 1 to 100,000 pulses per burst, and with a burst-repetition rate of 0.01 to 10,000 Hertz (Hz), are selectively applied to human, animal and plant cells, organs, tissues and molecules. The voltage-amplitude envelope of each pulse burst is a function of a random, irregular, or other like variable effective to provide a broad spectral density within the burst envelope. The variables are defined by mathematical functions that take into account signal to thermal noise ratio and Power SNR in specific target pathway structures. The waveforms are designed to modulate living cell growth, condition and repair. Particular applications of these signals include, but are not limited to, enhancing the effects of pharmacological, chemical, cosmetic and topical agents, prophylactic and wellness treatment of organs, muscles, joints, skin and hair, post surgical and traumatic wound repair, angiogenesis, improved blood perfusion, vasodilation, vasoconstriction, edema reduction, enhanced neovascularization, bone repair, tendon repair, ligament repair, organ regeneration and pain relief. The application of the within electromagnetic waveforms in conjunction with pharmacological, chemical, cosmetic and topical agents as applied to, upon or in human, animal and plant cells, organs, tissues and molecules can serve to enhance various effects of such compounds. A device according to the preamble of claim 1 is known from WO 2004108208.

According to an embodiment of the present invention a pulse burst envelope of higher spectral density can more efficiently couple to physiologically relevant dielectric pathways, such as cellular membrane receptors, ion binding to cellular enzymes, and general transmembrane potential changes. An embodiment according to the present invention increases the number of frequency components transmitted to relevant cellular pathways, resulting in a larger range of biophysical phenomena applicable to known healing mechanisms becoming accessible, including enhanced enzyme activity, growth factor release and cytokine release. By increasing burst duration and by applying a random, or other high spectral density envelope, to a pulse burst envelope of mono- or bi-polar rectangular or sinusoidal pulses that induce peak electric fields between $10^{-6}$ and 10 volts per centimeter (V/cm), and that satisfy detectability requirements according to SNR or Power SNR, a more efficient and greater effect could be achieved on biological healing processes applicable to both soft and hard tissues in humans, animals and plants resulting in enhancement of the effectiveness of pharmacological, chemical, cosmetic, and topical agents.

The present invention relates to known mechanisms of pharmacological, chemical, cosmetic and topical agents as applied to, upon or in human, animal and plant cells, organs, tissues and molecules. Specifically, the agents' efficacy depends upon arrival of optimal dosages of the agents to intended target pathway structures, which can be accomplished either via enhanced blood flow or enhanced chemical activity catalyzed by an increase in active enzymes during a relevant biochemical cascade. Electromagnetic fields can enhance blood flow and ion binding which affect the agents' activity. An advantageous result of using the present invention is that the quantity of an agent may be able to be reduced due to the agents enhanced effectiveness. It is an object of the present invention to provide an improved means to enhance and accelerate the intended effects, and improve efficacy as well as other effects of pharmacological, chemical, cosmetic and topical agents applied to, upon or in human, animal and plant cells, organs, tissues and molecules.

Another object of the present invention is that by applying a high spectral density voltage envelope as a modulating or pulse-burst defining parameter according to SNR and Power SNR requirements, power requirements for such increased duration pulse bursts can be significantly lower than that of shorter pulse bursts having pulses within the same frequency range; this results from more efficient matching of frequency components to a relevant cellular/molecular process. Accordingly, the advantages, of enhanced transmitted dosimetry to relevant dielectric pathways and of decreased power requirements are achieved.

Therefore, a need exists for an apparatus and a method that more effectively enhances and accelerates the intended effects, and improve efficacy as well as other bioeffective effects of pharmacological, chemical, cosmetic and topical agents applied to, upon or in human, animal and plant cells, organs, tissues and molecules.

## SUMMARY OF THE INVENTION

The present invention is defined in claim 1 and relates to enhancing effectiveness of pharmacological, chemical, cosmetic and topical agents used to treat living tissues, cells and molecules by providing a therapeutic, prophylactic and wellness apparatus for non-invasive pulsed electromagnetic treatment to enhance condition, repair and growth of living tissue in animals, humans and plants. A beneficial method not part of the invention operates to selectively change a bio-electromagnetic environment associated with cellular and tissue environments by using electromagnetic means such as EMF generators and applicator heads.

A flux path can be introduced to a selectable body region, comprising a succession of EMF pulses having a minimum width characteristic of at least 0.01 microseconds in a pulse burst envelope having between 1 and 100,000 pulses per burst, in which a voltage amplitude envelope of said pulse burst is defined by a randomly varying parameter in which an instantaneous minimum amplitude thereof is not smaller than a maximum amplitude thereof by a factor of one ten thousandth. Further, the repetition rate of such pulse bursts may vary from 0.01 to 10,000 Hertz. A mathematically definable parameter satisfying SNR and/or Power SNR detectability requirements in a target structure is employed to define the configuration of the pulse bursts.

Mathematically defined parameters are selected by considering the dielectric properties of the target pathway structure, and the ratio of the induced electric field amplitude with respect to voltage due to thermal noise or other baseline cellular activity.

A method not part of the invention is for treating living cells and tissue by electromagnetically modulating sensitive regulatory processes at a cell membrane and at junctional interfaces between cells, using waveforms configured to satisfy SNR and Power SNR detectability requirements in a target pathway structure.

A preferred embodiment according to the present invention utilizes a Power Signal to Noise Ratio ("Power SNR") approach to configure bioeffective waveforms and incorporates miniaturized circuitry and lightweight flexible coils. This advantageously allows a device that utilizes a Power SNR approach, miniaturized circuitry, and lightweight flexible coils, to be completely portable and if desired to be constructed as disposable and if further desired to be constructed as implantable.

Specifically, broad spectral density bursts of electromagnetic waveforms, configured to achieve maximum signal power within a bandpass of a biological target, are selectively applied to target pathway structures such as tissues, to enhance effectiveness of pharmacological, chemical, cosmetic and topical agents. Waveforms are selected using a unique amplitude/power comparison with that of thermal noise in a target pathway structure. Signals comprise bursts of at least one of sinusoidal, rectangular, chaotic and random wave shapes, have frequency content in a range of about 0.01 Hz to about 100 MHz at about 1 to about 100,000 bursts per second, and have a burst repetition rate from about 0.01 to about 1000 bursts/second. Peak signal amplitude at a target pathway structure such as organs, cells, tissues, and molecules, lies in a range of about 1 $\mu$V/cm to about 100 mV/cm. Each signal burst envelope may be a random function providing a means to accommodate different electromagnetic characteristics of enhancing bioeffective processes. A preferred embodiment according to the present invention comprises about 0.1 to about 100 millisecond pulse burst comprising about 1 to about 200 microsecond symmetrical or asymmetrical pulses repeating at about 0.1 to about 100 kilohertz within the burst. The burst envelope is a modified 1/f function and is applied at random repetition rates between about 0.1 and about 1000 Hz. Fixed repetition rates can also be used between about 0.1 Hz and about 1000 Hz. An induced electric field from about 0.001 mV/cm to about 100 mV/cm is generated. Another embodiment according to the present invention comprises an about 0.01 millisecond to an about 10 millisecond burst of high frequency sinusoidal waves, such as 27.12 MHz, repeating at about 1 to about 100 bursts per second. An induced electric field from about 0.001 mV/cm to about 100 mV/cm is generated. Resulting waveforms can be delivered via inductive or capacitive coupling.

## DISCLOSURE OF INVENTION

It is an object of the present invention to provide modulation of electromagnetically sensitive regulatory processes at the cell membrane and at junctional interfaces between cells.

It is another object of the present invention to enhance effectiveness of pharmacological, chemical, cosmetic and topical agents by configuring a power spectrum of a waveform by mathematical simulation by using signal to noise ratio ("SNR") analysis to configure a waveform optimized to modulate angiogensis and neovascualarization, then coupling the configured waveform using a generating device such as ultra lightweight wire coils that are powered by a waveform configuration device such as miniaturized electronic circuitry.

It is another object of the present invention to modulate angiogenesis and neovascularization by evaluating Power SNR at any target pathway structure such as molecules, cells, tissues and organs to enhance effectiveness of pharmacological, chemical, cosmetic and topical agents, by using any input waveform, even if electrical equivalents are non-linear as in a Hodgkin-Huxley membrane model.

It is another object of the present invention to provide an apparatus that incorporates use of Power SNR to regulate and adjust electromagnetic therapy treatment to enhance effectiveness of pharmacological, chemical, cosmetic and topical agents.

It is another object of the present invention to provide an apparatus for enhancing effectiveness of pharmacological, chemical, cosmetic and topical agents using electromagnetic fields selected by optimizing a power spectrum of a waveform to be applied to a biochemical target pathway structure to enable modulation of angiogenesis and neovascularization within molecules, cells, tissues and organs.

It is another object of the present invention to significantly lower peak amplitudes and shorter pulse duration by matching via Power SNR, a frequency range in a signal to frequency response and sensitivity of a target pathway structure such as a molecule, cell, tissue, and organ thereby enabling modulation of angiogenesis and neovascularization for enhancing effectiveness of pharmacological, chemical, cosmetic and topical agents.

It is a further object of the present invention to provide an apparatus for application of electromagnetic waveforms, to be used in conjunction with pharmacological, chemical, cosmetic and topical agents applied to, upon or in human, animal and plant cells, organs, tissues and molecules so that bioeffective processes of such compounds can be enhanced.

A method not part of the invention is disclosed to enhance effectiveness of pharmacological, chemical, cosmetic and topical agents for therapeutic, prophylactic and wellness ends.

A method not part of the invention is disclosed for treatment of organs, muscles, joints, skin and hair using EMF in conjunction with pharmacological, chemical, cosmetic and topical agents to improve the agents' effectiveness.

A method not part of the invention is disclosed for treatment of organs, muscles, joints, skin and hair using EMF in conjunction with pharmacological, chemical, cosmetic and topical agents to enhance wellness.

A method not part of the invention is disclosed in which electromagnetic waveforms are configured according to SNR and Power SNR detectability requirements in a target pathway structure.

A method not part of the invention is disclosed for electromagnetic treatment comprising a broadband, high spectral density electromagnetic field.

A method not part of the invention is disclosed of enhancing soft tissue and hard tissue repair by using EMF in conjunction with pharmacological, chemical, cosmetic and topical agents.

A method not part of the invention is disclosed to enhance effectiveness of pharmacological, chemical, cosmetic and topical agents by increasing blood flow to affected tissues by using electromagnetic treatment to modulate angiogenesis.

A method not part of the invention is disclosed to increase blood flow for enhancing effectiveness of pharmacological, chemical, cosmetic and topical agents that regulate viability, growth, and differentiation of implanted cells, tissues and organs.

A method not part of the invention is disclosed to treat cardiovascular diseases by modulating angiogensis and increasing blood flow to enhance effectiveness of pharmacological, chemical, cosmetic and topical agents.

A method not part of the invention is disclosed that increases physiological effectiveness of pharmacological, chemical, cosmetic and topical agents by improving micro-vascular blood perfusion and reduced transudation.

A method not part of the invention is disclosed to increase blood flow to enhance effectiveness of pharmacological, chemical, cosmetic and topical agents used for treating maladies of bone and hard tissue.

A method not part of the invention is disclosed to increase blood flow to enhance effectiveness of pharmacological, chemical, cosmetic and topical agents used for treating edema and swelling of soft tissue.

A method not part of the invention is disclosed to increase blood flow to enhance effectiveness of pharmacological, chemical, cosmetic and topical agents used for repairing damaged soft tissue.

A method not part of the invention is disclosed to increase blood flow to damaged tissue by modulation of vasodilation and stimulating neovascularization whereby enhanced effectiveness of pharmacological, chemical, cosmetic and topical agents is achieved.

It is a further object of the present invention to provide an electromagnetic treatment apparatus wherein the apparatus operates using reduced power levels.

It is a yet further object of the present invention to provide an electromagnetic treatment apparatus wherein the apparatus is inexpensive, portable, and produces reduced electromagnetic interference.

The above and yet other objects and advantages of the present invention will become apparent from the hereinafter set forth Brief Description of the Drawings, Detailed Description of the Invention, and Claims appended herewith.

## BRIEF DESCRIPTION OF THE DRAWINGS

Preferred embodiments of the present invention will be described below in more detail, with reference to the accompanying drawings:

Figure 1 is a flow diagram of a method not part of the invention for enhancing effectiveness of pharmacological, chemical, cosmetic and topical agents used to treat living tissues, cells and molecules according to an embodiment of the present invention;

Figure 2 is a view of control circuitry and electrical coils applied to a knee joint according to a preferred embodiment of the present invention;

Figure 3 is a block diagram of miniaturized circuitry according to a preferred embodiment of the present invention;

Figures 4A is a line drawing of a wire coil such as an inductor according to a preferred embodiment of the present invention;

Figures 4B is a line drawing of a flexible magnetic wire according to a preferred embodiment of the present invention;

Figure 5 depicts a waveform delivered to a target pathway structure such as a molecule, cell, tissue or organ according to a preferred embodiment of the present invention;

Figure 6 is a view of a positioning device such as a wrist support according to a preferred embodiment of the present invention;

Figure 7 is a view of a positioning device such as a mattress pad according to a preferred embodiment of the present invention;

Figure 8 is a graph illustrating effects of increased burst duration according to an embodiment of the present invention; and

Figure 9 is a graph illustrating an increase in skin blood perfusion achieved according to an embodiment of the present invention.

## MODES FOR CARRYING OUT THE INVENTION

An embodiment according to the present invention provides a higher spectral density to a pulse burst envelope resulting in enhanced effectiveness of therapy upon relevant dielectric pathways, such as, cellular membrane receptors, ion binding to cellular enzymes and general transmembrane potential changes. An embodiment according to the present invention increases the number of frequency components transmitted to relevant cellular pathways, thereby providing access to a larger range of biophysical phenomena applicable to known healing mechanisms, for example modulation of growth factor and cytokine release, and ion binding at regulatory molecules. By applying a random, or other high spectral density envelope, according to a mathematical model defined by SNR or Power SNR in a transduction pathway, to a pulse burst envelope of mono- or bi-polar rectangular or sinusoidal pulses inducing peak electric fields between $10^{-6}$ and 10 volts per centimeter (V/cm), a greater effect could be accomplished on biological healing processes applicable to both soft and hard tissues thereby enhancing effectiveness of pharmacological, chemical, cosmetic and topical agents.

An advantageous result of the present invention, is that by applying a high spectral density voltage envelope as the modulating or pulse-burst defining parameter, according to a mathematical model defined by SNR or Power SNR in a transduction pathway, the power requirement for such amplitude modulated pulse bursts can be significantly lower than that of an unmodulated pulse burst containing pulses within the same frequency range. Accordingly, the advantages of enhanced transmitted dosimetry to the relevant dielectric target pathways and of decreased power requirement are achieved.

An additional advantage of the present invention relates to enhanced effectiveness of pharmacological, chemical, cosmetic and topical agents as applied to, upon or on human, animal and plant cells, organs, tissues and molecules by accelerating the agents intended effects and improving efficacy.

Induced time-varying currents from PEMF or PRF devices flow in a target pathway structure such as a molecule, cell, tissue, and organ, and it is these currents that are a stimulus to which cells and tissues can react in a physiologically meaningful manner. The electrical properties of a target pathway structure affect levels and distributions of induced current. Molecules, cells, tissue, and organs are all in an induced current pathway such as cells in a gap junction contact. Ion or ligand interactions at binding sites on macromolecules that may reside on a membrane surface are voltage dependent processes, for example electrochemical, that can respond to an induced electromagnetic field ("E"). Induced current arrives at these sites via a surrounding ionic medium. The presence of cells in a current pathway causes an induced current ("J") to decay more rapidly with time ("J(t)"). This is due to an added electrical impedance of cells from membrane capacitance and time constants of binding and other voltage sensitive membrane processes such as membrane transport.

Equivalent electrical circuit models representing various membrane and charged interface configurations have been derived. For example, in Calcium ("$Ca^{2+}$") binding, the change in concentration of bound $Ca^{2+}$ at a binding site due to induced E may be described in a frequency domain by an impedance expression such as:

$$Z_b(\omega) = R_{ion} + \frac{1}{i\,\omega\,C_{ion}}$$

which has the form of a series resistance-capacitance electrical equivalent circuit. Where $\omega$ is angular frequency defined as $2\pi f$, where f is frequency, $i = -1^{1/2}$, $Z_b(\omega)$ is the binding impedance, and $R_{ion}$ and $C_{ion}$ are equivalent binding resistance and capacitance of an ion binding pathway. The value of the equivalent binding time constant, $\tau_{ion} = R_{ion}C_{ion}$, is related to a ion binding rate constant, $k_b$, via $\tau_{ion} = R_{ion}C_{ion} = 1/k_b$. Thus, the characteristic time constant of this pathway is determined by ion binding kinetics.

Induced E from a PEMF or PRF signal can cause current to flow into an ion binding pathway and affect the number of $Ca^{2+}$ ions bound per unit time. An electrical equivalent of this is a change in voltage across the equivalent binding capacitance $C_{ion}$, which is a direct measure of the change in electrical charge stored by $C_{ion}$. Electrical charge is directly proportional to a surface concentration of $Ca^{2+}$ ions in the binding site, that is storage of charge is equivalent to storage of ions or other charged species on cell surfaces and junctions. Electrical impedance measurements, as well as direct kinetic analyses of binding rate constants, provide values for time constants necessary for configuration of a PMF waveform to match a bandpass of target pathway structures. This allows for a required range of frequencies for any given induced E waveform for optimal coupling to target impedance, such as bandpass.

Ion binding to regulatory molecules is a frequent EMF target, for example $Ca^{2+}$ binding to calmodulin ("CaM"). Use of this pathway is based upon acceleration of tissue repair, for example bone repair, wound repair, hair repair, and repair of molecules, cells, tissues, and organs that involves modulation of growth factors released in various stages of repair. Growth factors such as platelet derived growth factor ("PDGF"), fibroblast growth factor ("FGF"), and epidermal growth factor ("EGF") are all involved at an appropriate stage of healing. Angiogenesis and neovascularization are also integral to tissue growth and repair and can be modulated by PMF. All of these factors are Ca/CaM-dependent.

Utilizing a Ca/CaM pathway a waveform can be configured for which induced power is sufficiently above background thermal noise power. Under correct physiological conditions, this waveform can have a physiologically significant bioeffect.

Application of a Power SNR model to Ca/CaM requires knowledge of electrical equivalents of $Ca^{2+}$ binding kinetics at CaM. Within first order binding kinetics, changes in concentration of bound $Ca^{2+}$ at CaM binding sites over time may be characterized in a frequency domain by an equivalent binding time constant, $\tau_{ion} = R_{ion}\text{-}C_{ion}$, where $R_{ion}$ and $C_{ion}$ are equivalent binding resistance and capacitance of the ion binding pathway. $\tau_{ion}$ is related to a ion binding rate constant, $k_b$, via $\tau_{ion} = R_{ion}\text{-}C_{ion} = 1/k_b$. Published values for $k_b$ can then be employed in a cell array model to evaluate SNR by comparing voltage induced by a PRF signal to thermal fluctuations in voltage at a CaM binding site. Employing numerical values for PMF response, such as $V_{max}=6.5\text{x}10^{-7}$ sec$^{-1}$, $[Ca^{2+}] = 2.5$pM, $K_D=30\mu$M, $[Ca^{2+}CaM] = K_D([Ca^{2+}] + [CaM])$, yields $k_b=665$ sec$^{-1}$ ($\tau_{ion}$ = 1.5 msec). Such a value for $\tau_{ion}$ can be employed in an electrical equivalent circuit for ion binding while power SNR analysis can be performed for any waveform structure.

According to an embodiment of the present invention a mathematical model for example a mathematical equation and or a series of mathematical equations can be configured to assimilate that thermal noise is present in all voltage dependent processes and represents a minimum threshold requirement to establish adequate SNR. For example a mathematical model that represents a minimum threshold requirement to establish adequate SNR can be configured to include power spectral density of thermal noise such that power spectral density, $S_n(\omega)$, of thermal noise can be expressed as:

$$S_n(\omega) = 4kT\,Re[Z_M(x,\omega)]$$

where $Z_M(x,\omega)$ is electrical impedance of a target pathway structure, x is a dimension of a target pathway structure and Re denotes a real part of impedance of a target pathway structure. $Z_M(x,\omega)$ can be expressed as:

$$Z_M(x,\omega) = \left[\frac{R_e + R_i + R_g}{\gamma}\right]\tanh(\gamma x)$$

This equation clearly shows that electrical impedance of the target pathway structure, and contributions from extracellular fluid resistance ("$R_e$"), intracellular fluid resistance ("$R_i$") and intermembrane resistance ("Rg") which are electrically connected to a target pathway structures, all contribute to noise filtering.

A typical approach to evaluation of SNR uses a single value of a root mean square (RMS) noise voltage. This is calculated by taking a square root of an integration of $S_n(\omega) = 4kT\,Re[Z_M(x,\omega)]$ over all frequencies relevant to either complete membrane response, or to bandwidth of a target pathway structure. SNR can be expressed by a ratio:

$$SNR = \frac{|V_M(\omega)|}{RMS}$$

where $|V_M(\omega)|$ is maximum amplitude of voltage at each frequency as delivered by a chosen waveform to the target pathway structure.

An embodiment according to the present invention comprises a pulse burst envelope having a high spectral density, so that the effect of therapy upon the relevant dielectric pathways, such as, cellular membrane receptors, ion binding to cellular enzymes and general transmembrane potential changes, is enhanced. Accordingly by increasing a number of frequency components transmitted to relevant cellular pathways, a large range of biophysical phenomena, such as modulating growth factor and cytokine release and ion binding at regulatory molecules, applicable to known tissue growth mechanisms is accessible. According to an embodiment of the present invention applying a random, or other high spectral density envelope, to a pulse burst envelope of mono- or bi-polar rectangular or sinusoidal pulses inducing peak electric fields between about $10^{-8}$ and about 100 V/cm, produces a greater effect on biological healing processes applicable to both soft and hard tissues.

According to yet another embodiment of the present invention by applying a high spectral density voltage envelope as a modulating or pulse-burst defining parameter, power requirements for such amplitude modulated pulse bursts can be significantly lower than that of an unmodulated pulse burst containing pulses within a similar frequency range. This is due to a substantial reduction in duty cycle within repetitive burst trains brought about by imposition of an irregular, and preferably random, amplitude onto what would otherwise be a substantially uniform pulse burst envelope. Accordingly, the dual advantages, of enhanced transmitted dosimetry to the relevant dielectric pathways and of decreased power requirement are achieved.

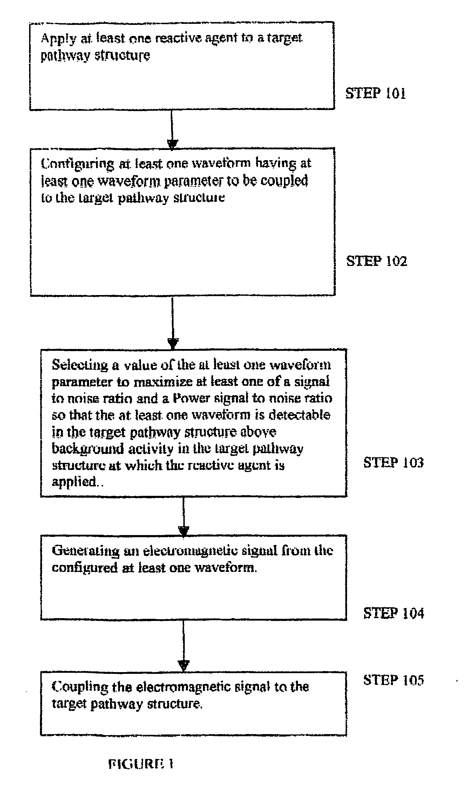

Referring to Figure 1, wherein Figure 1 is a flow diagram of a method not part of the present invention, for enhancing effectiveness of pharmacological, chemical, cosmetic and topical agents used to treat stem cells, tissues, cells, organs, and molecules by delivering electromagnetic signals that can be pulsed, to target pathway structures such as ions and ligands of animals and humans, for therapeutic and prophylactic purposes. Target pathway structures can also include but are not limited to stem cells, tissues, cells, organs, and molecules. Enhancing effectiveness of pharmacological, chemical, cosmetic and topical agents includes but is not limited to increased absorption rate, decreased effective dosages, faster delivery rates at an organism level; and increased binding kinetics and transport kinetics level at a molecular and cellular level.

At least one reactive agent is applied to a target pathway structure (Step 101). Reactive agents include but are not limited to pharmacological agents, chemical agents, cosmetic agents, topical agents, and genetic agents. Reactive agents can be ingested, applied topically, applied intravenously, intramuscularly, or by any other manner known within the medical community that causes interaction of substances with a target pathway structure, such as iontophoresis, X and light radiation, and heat. Pharmacological agents include but are not limited to antibiotics, growth factors, chemotherapeutic agents, antihistamines, Angiotensin inhibitors, beta blockers, statins, and anti-inflammatory drugs. Chemical agents include but are not limited to hydrogen peroxide, betadine, and alcohol. Topical agents include but are not limited to antibiotics, creams, retinol, benzoyl peroxide, tolnaftate, menthol, emollients, oils, lanolin, squalene, aloe vera, anti-oxidants, fatty acid, fatty acid ester, cod liver oil, alpha-tocopherol, petroleum, hydrogenated polybutene, vitamin A, vitamin E, topical proteins, and collagens. Cosmetic agents include but are not limited to make-up, eye-liner, and blush. Genetic agents include but are not limited to genes, DNA, and chromosomes.

Configuring at least one waveform having at least one waveform parameter to be coupled to the target pathway structure such as ions and ligands (Step 102).

The at least one waveform parameter is selected to maximize at least one of a signal to noise ratio and a Power Signal to Noise ratio in a target pathway structure so that a waveform is detectable in the target pathway structure above its background activity (Step 102) such as baseline thermal fluctuations in voltage and electrical impedance at a target pathway structure that depend upon a state of a cell and tissue, that is whether the state is at least one of resting, growing, replacing, and responding to injury to produce physiologically beneficial results. To be detectable in the target pathway structure the value of said at least one waveform parameter is chosen by using a constant of said target pathway structure to evaluate at least one of a signal to noise ratio, and a Power signal to noise ratio, to compare voltage induced by said at least one waveform in said target pathway structure to baseline thermal fluctuations in voltage and electrical impedance in said target pathway structure whereby bioeffective modulation occurs in said target pathway structure by said at least one waveform by maximizing said at least one of signal to noise ratio and Power signal to noise ratio, within a bandpass of said target pathway structure.

A preferred embodiment of a generated electromagnetic signal is comprised of a burst of arbitrary waveforms having at least one waveform parameter that includes a plurality of frequency components ranging from about 0.01 Hz to about 100 MHz wherein the plurality of frequency components satisfies a Power SNR model (Step 103). A repetitive electromagnetic signal can be generated for example inductively or capacitively, from said configured at least one waveform (Step 104). The electromagnetic signal can also be non-repetitive. The electromagnetic signal is coupled to a target pathway structure such as ions and ligands by output of a coupling device such as an electrode or an inductor, placed in close proximity to the target pathway structure (Step 105). Coupling of the electromagnetic signal to a target pathway structure can occur adjunctively, for example at any time prior to applying a reactive agent, at the same time a reactive agent is being applied, or after the time a reactive agent has been applied. The coupling enhances blood flow and modulation of binding of ions and ligands to regulatory molecules in molecules, tissues, cells, and organs thereby enhancing the reactive agents' bioeffectiveness.

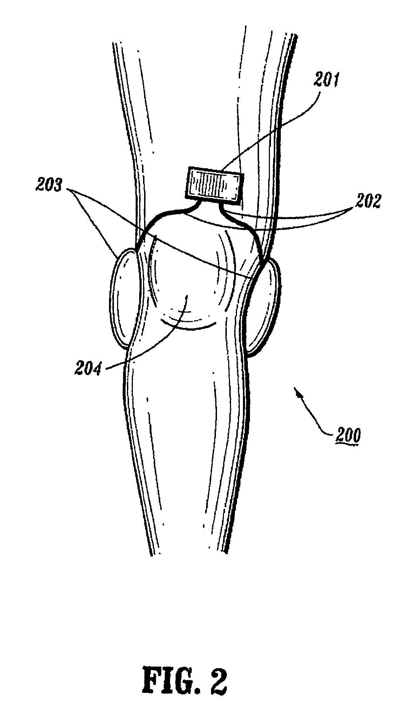

Figure 2 illustrates a preferred embodiment of an apparatus according to the present invention. The apparatus is self-contained, lightweight, and portable. A miniature control circuit 201 is coupled to an end of at least one connector 202 such as wire however the control circuit can also operate wirelessly. The opposite end of the at least one connector is coupled to a generating device such as an electrical coil 203. The miniature control circuit 201 is constructed in a manner that applies a mathematical model that is used to configure waveforms. The configured waveforms have to satisfy Power SNR so that for a given and known target pathway structure, it is possible to choose waveform parameters that satisfy Power SNR so that a waveform produces physiologically beneficial results, for example bioeffective modulation, and is detectable in the target pathway structure above its background activity. A preferred embodiment according to the present invention applies a mathematical model to induce a time-varying magnetic field and a time-varying electric field in a target pathway structure such as ions and ligands, comprising about 0.1 to about 100 msec bursts of about 1 to about 100 microsecond rectangular pulses repeating at about 0.1 to about 100 pulses per second. Peak amplitude of the induced electric field is between about 1 uV/cm and about 100 mV/cm, varied according to a modified 1/f function where f = frequency. A waveform configured using a preferred embodiment according to the present invention may be applied to a target pathway structure such as ions and ligands for a preferred total exposure time of under 1 minute to 240 minutes daily. However other exposure times can be used. Waveforms configured by the miniature control circuit 201 are directed to a generating device 203 such as electrical coils via connector 202. The generating device 203 delivers a pulsing magnetic field that can be used to provide treatment to a target pathway structure such as tissue. The miniature control circuit applies a pulsing magnetic field for a prescribed time and can automatically repeat applying the pulsing magnetic field for as many applications as are needed in a given time period, for example 10 times a day. The miniature control circuit can be configured to be programmable applying pulsing magnetic fields for any time repetition sequence. A preferred embodiment according to the present invention can enhance the pharmacological, chemical, cosmetic and topical agents' effectiveness by being incorporated into a positioning device 204, for example a bed. Coupling a pulsing magnetic field to a target pathway structure such as ions and ligands, therapeutically and prophylactically reduces inflammation thereby advantageously reducing pain, promoting healing in targeted areas, and enhancing interactions of pharmacological, chemical, cosmetic and topical agents with a target pathway structure. When electrical coils are used as the generating device 203, the electrical coils can be powered with a time varying magnetic field that induces a time varying electric field in a target pathway structure according to Faraday's law. An electromagnetic signal generated by the generating device 203 can also be applied using electrochemical coupling, wherein electrodes are in direct contact with skin or another outer electrically conductive boundary of a target pathway structure. Yet in another embodiment according to the present invention, the electromagnetic signal generated by the generating device 203 can also be applied using electrostatic coupling wherein an air gap exists between a generating device 203 such as an electrode and a target pathway structure such as ions and ligands. An advantage of the preferred embodiment according to the present invention is that its ultra lightweight coils and miniaturized circuitry allow for use with common physical therapy treatment modalities and at any for which growth, pain relief, and tissue and organ healing is desired. An advantageous result of application of the preferred embodiment according to the present invention is that tissue growth, repair, and maintenance can be accomplished and enhanced anywhere and at anytime, for example while driving a car or watching television. Yet another advantageous result of application of the preferred embodiment is that growth, repair, and maintenance of molecules, cells, tissues, and organs can be accomplished and enhanced anywhere and at anytime, for example while driving a car or watching television.

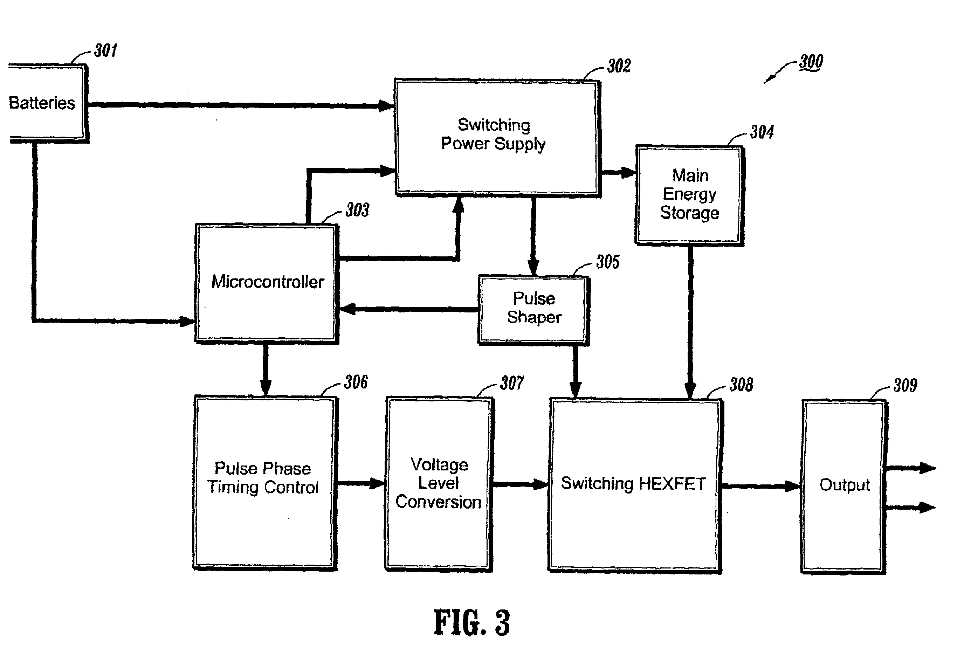

Figure 3 depicts a block diagram of a preferred embodiment according to the present invention of a miniature control circuit 300. The miniature control circuit 300 produces waveforms that drive a generating device such as wire coils described above in Figure 2. The miniature control circuit can be activated by any activation means such as an on/off switch. The miniature control circuit 300 has a power source such as a lithium battery 301. A preferred embodiment of the power source has an output voltage of 3.3 V but other voltages can be used. In another embodiment according to the present invention the power source can be an external power source such as an electric current outlet such as an AC/DC outlet, coupled to the present invention for example by a plug and wire. A switching power supply 302 controls voltage to a micro-controller 303. A preferred embodiment of the micro-controller 303 uses an 8 bit 4 MHz micro-controller 303 but other bit MHz combination microcontrollers may be used. The switching power supply 302 also delivers current to storage capacitors 304. A preferred embodiment of the present invention uses storage capacitors having a 220 uF output but other outputs can be used. The storage capacitors 304 allow high frequency pulses to be delivered to a coupling device such as inductors (Not Shown). The micro-controller 303 also controls a pulse shaper 305 and a pulse phase timing control 306. The pulse shaper 305 and pulse phase timing control 306 determine pulse shape, burst width, burst envelope shape, and burst repetition rate. An integral waveform generator, such as a sine wave or arbitrary number generator can also be incorporated to provide specific waveforms. A voltage level conversion sub-circuit 307 controls an induced field delivered to a target pathway structure. A switching Hexfet 308 allows pulses of randomized amplitude to be delivered to output 309 that routes a waveform to at least one coupling device such as an inductor. The micro-controller 303 can also control total exposure time of a single treatment of a target pathway structure such as a molecule, cell, tissue, and organ. The miniature control circuit 300 can be constructed to be programmable and apply a pulsing magnetic field for a prescribed time and to automatically repeat applying the pulsing magnetic field for as many applications as are needed in a given time period, for example 10 times a day. A preferred embodiment according to the present invention uses treatments times of about 10 minutes to about 30 minutes.

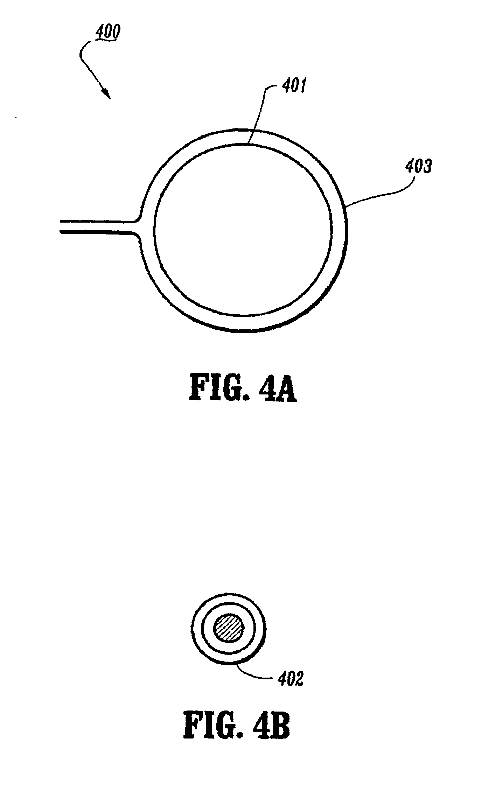

Referring to Figures 4A and 4B a preferred embodiment according to the present invention of a coupling device 400 such as an inductor is shown. The coupling device 400 can be an electric coil 401 wound with single or multistrand flexible wire 402 however solid wire can also be used. In a preferred embodiment according to the present invention the wire is made of copper but other materials can be used. The multistrand flexible magnetic wire 402 enables the electric coil 401 to conform to specific anatomical configurations such as a limb or joint of a human or animal. A preferred embodiment of the electric coil 401 comprises about 1 to about 1000 turns of about 0.01 mm to about 0.1 mm diameter at least one of single magnet wire and multistrand magnet wire, wound on an initially circular form having an outer diameter between about 2.5 cm and about 50 cm but other numbers of turns and wire diameters can be used. A preferred embodiment of the electric coil 401 can be encased with a non-toxic PVC mould 403 but other non-toxic moulds can also be used. The electric coil can also be incorporated in dressings, bandages, garments, and other structures typically used tor wound treatment.

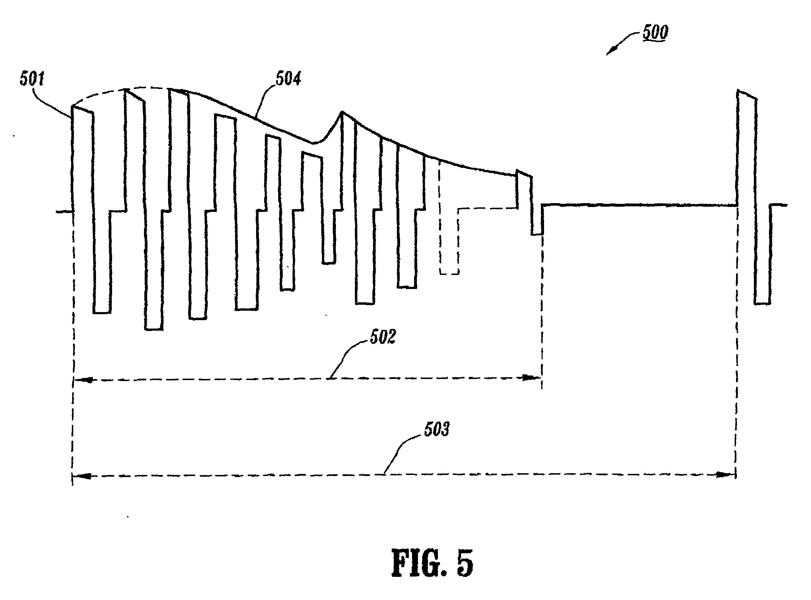

Referring to Figure 5 an embodiment according to the present invention of a waveform 500 is illustrated. A pulse 501 is repeated within a burst 502 that has a finite duration 503. The duration 503 is such that a duty cycle which can be defined as a ratio of burst duration to signal period is between about 1 to about $10^{-5}$. A preferred embodiment according to the present invention utilizes pseudo rectangular 10 microsecond pulses for pulse 501 applied in a burst 502 for about 10 to about 50 msec having a modified 1/f amplitude envelope 504 and with a finite duration 503 corresponding to a burst period of between about 0.1 and about 10 seconds, but other waveforms, envelopes, and burst periods that follow a mathematical model such as SNR and Power SNR, may be used.

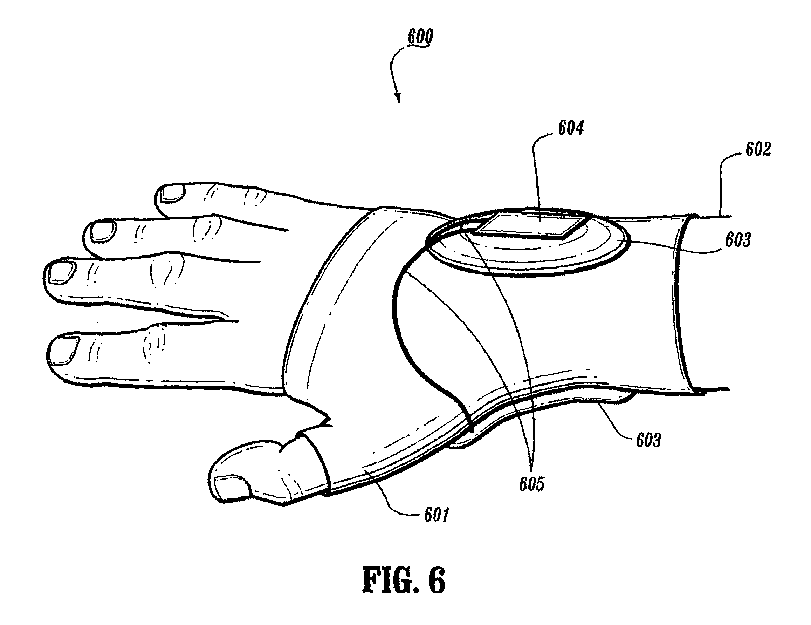

Figure 6 illustrates a preferred embodiment according to the present invention of a positioning device such as a wrist support. A positioning device 600 such as a wrist support 601 is worn on a human wrist 602. The positioning device can be constructed to be portable, can be constructed to be disposable, and can be constructed to be implantable. The positioning device can be used in combination with the present invention in a plurality of ways, for example incorporating the present invention into the positioning device for example by stitching, affixing the present invention onto the positioning device for example by Velcro®, and holding the present invention in place by constructing the positioning device to be elastic.

In another embodiment according to the present invention, the present invention can be constructed as a stand-alone device of any size with or without a positioning device, to be used anywhere for example at home, at a clinic, at a treatment center, and outdoors. The wrist support 601 can be made with any anatomical and support material, such as neoprene. Coils 603 are integrated into the wrist support 601 such that a signal configured according to the present invention, for example the waveform depicted in Figure 5, is applied from a dorsal portion that is the top of the wrist to a plantar portion that is the bottom of the wrist. Micro-circuitry 604 is attached to the exterior of the wrist support 601 using a fastening device such as Velcro® (Not Shown). The micro-circuitry is coupled to one end of at least one connecting device such as a flexible wire 605. The other end of the at least one connecting device is coupled to the coils 603. Other embodiments according to the present invention of the positioning device include knee, elbow, lower back, shoulder, other anatomical wraps, and apparel such as garments, fashion accessories, and footware.

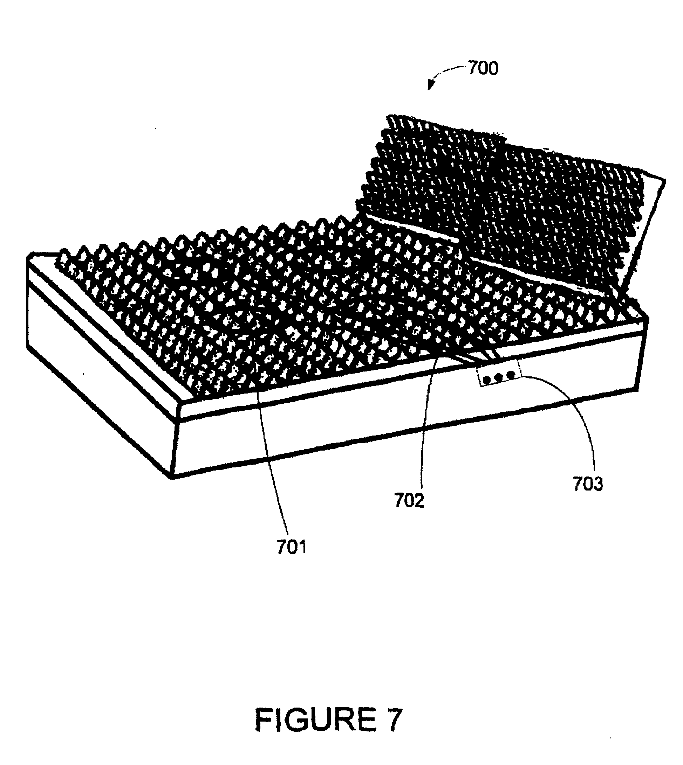

Referring to Figure 7 an embodiment according to the present invention of an electromagnetic treatment apparatus integrated into a mattress pad 700 is illustrated. A mattress can also be used. Several lightweight flexible coils 701 are integrated into the mattress pad. The lightweight flexible coils can be constructed from fine flexible conductive wire, conductive thread, and any other flexible conductive material. The flexible coils are connected to at least one end of at least one wire 702. However, the flexible coils can also be configured to be directly connected to circuitry 703 or wireless. Lightweight miniaturized circuitry 703 that configures waveforms according to an embodiment of the present invention, is attached to at least one other end of said at least on wire. When activated the lightweight miniaturized circuitry 703 configures waveforms that are directed to the flexible coils (701) to create PEMF signals that are coupled to a target pathway structure.

## Example 1

An embodiment according to the present invention for EMF signal configuration has been used on calcium dependent myosin phosphorylation in a standard enzyme assay. This enzyme pathway is known to enhance the effects of pharmacological, chemical, cosmetic and topical agents as applied to, upon or in human, animal and plant cells, organs, tissues and molecules. The reaction mixture was chosen for phosphorylation rate to be linear in time for several minutes, and for sub-saturation $Ca^{2+}$ concentration. This opens the biological window for $Ca^{2+}$/CaM to be EMF-sensitive, as happens in an injury or with the application of pharmacological, chemical, cosmetic and topical agents as applied to, upon or in human, animal and plant cells, organs, tissues and molecules. Experiments were performed using myosin light chain ("MLC") and myosin light chain kinase ("MLCK") isolated from turkey gizzard. A reaction mixture consisted of a basic solution containing 40 mM Hepes buffer, pH 7.0; 0.5 mM magnesium acetate; 1 mg/ml bovine serum albumin, 0.1% (w/v) Tween 80; and 1 mM EGTA. Free $Ca^{2+}$ was varied in the 1-7 μM range. Once $Ca^{2+}$ buffering was established, freshly prepared 70 nM CaM, 160 nM MLC and 2 nM MLCK were added to the basic solution to form a final reaction mixture.

The reaction mixture was freshly prepared daily for each series of experiments and was aliquoted in 100 μL portions into 1.5 ml Eppendorf tubes. All Eppendorf tubes containing reaction mixture were kept at 0°C then transferred to a specially designed water bath maintained at 37 ± 0.1°C by constant perfusion of water prewarmed by passage through a Fisher Scientific model 900 heat exchanger. Temperature was monitored with a thermistor probe such as a Cole-Parmer model 8110-20, immersed in one Eppendorf tube during all experiments. Reaction was initiated with 2.5 μM 32P ATP, and was stopped with Laemmli Sample Buffer solution containing 30 μM EDTA. A minimum of five blank samples were counted in each experiment. Blanks comprised a total assay mixture minus one of the active components $Ca^{2+}$, CaM, MLC or MLCK. Experiments for which blank counts were higher than 300 cpm were rejected. Phosphorylation was allowed to proceed for 5 min and was evaluated by counting $^{32}P$ incorporated in MLC using a TM Analytic model 5303 Mark V liquid scintillation counter.

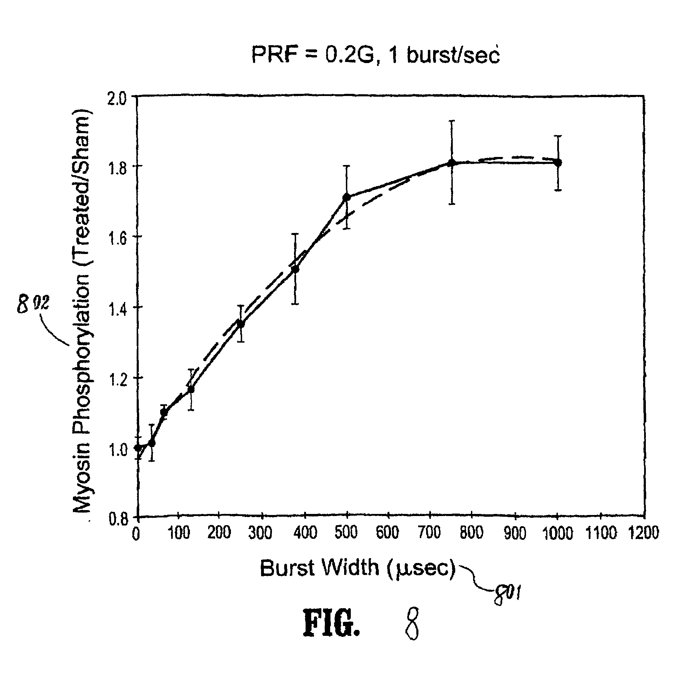

The signal comprised repetitive bursts of a high frequency waveform. Amplitude was maintained constant at 0.2G and repetition rate was 1 burst/sec for all exposures. Burst duration varied from 65 μsec to 1000 μsec based upon projections of mathematical analysis of the instant invention which showed that optimal Power SNR would be achieved as burst duration approached 500 μsec. The results are shown in Figure 8 wherein burst width 801 in μsec is plotted on the x-axis and Myosin Phosphorylation 802 as treated/sham is plotted on the y-axis. It can be seen that the PMF effect on $Ca^{2+}$ binding to CaM approaches its maximum at approximately 500 μsec, just as illustrated by the Power SNR model.

These results confirm that an EMF signal, configured according to an embodiment of the present invention, would maximally increase the effect of pharmacological, chemical, cosmetic and topical agents as applied to, upon or in human, animal and plant cells, organs, tissues and molecules for burst durations sufficient to achieve optimal Power SNR for a given magnetic field amplitude.

## Example 2

This study determined to what extent treatment with pulsed electromagnetic frequency ("PEMF") waveforms affects blood perfusion in a treated region. All testing was done in a temperature controlled room (23 to 24° C) with the subject seated on a comfortable easy chair. On each arm a non-metallic laser Doppler probe was affixed with double-sided tape to a medial forearm site approximately 5cm distal to the antecubital space. A temperature sensing thermistor for surface temperature measurements was placed approximately 1cm distal to the outer edge of the probes and secured with tape. A towel was draped over each forearm to diminish the direct effects of any circulating air currents. With the subject resting comfortably, the skin temperature of each arm was monitored. During this monitoring interval the excitation coil for producing the PEMF waveform according to the instant invention was positioned directly above the Laser Doppler probe of the right forearm at a vertical distance of approximately 2cm from the skin surface. When the monitored skin temperature reached a steady state value, the data acquisition phase was begun. This consisted of a 20 minute baseline interval followed by a 45 minute interval in which the PEMF waveform was applied.

Skin temperature was recorded at five minute intervals during the entire protocol. Blood perfusion signals as determined with the Laser Doppler Flowmeter ("LDF") were continuously displayed on a chart recorder and simultaneously acquired by a computer following analog to digital conversion. The LDF signals were time averaged by the computer during each contiguous five minute interval of measurement to produce a single averaged perfusion value for each interval. At the end of the procedure the relative magnetic field strength at the skin site was measured with a 1 cm diameter loop which was coupled to a specially designed and calibrated metering system.

For each subject the baseline perfusion for the treated arm and the control arm was determined as the average during the 20 minute baseline interval. Subsequent perfusion values, following the start of PEMF treatment, was expressed as a percentage of this baseline. Comparison between the treated and control arms were done using analysis of variance with arm (treated vs. control) as the grouping variables and with time as a repeated measure.

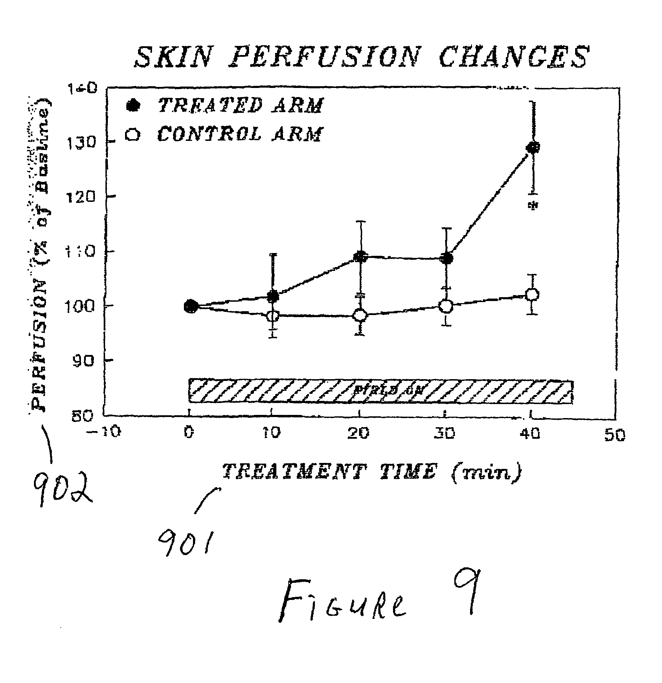

Figure 9 summarizes the time course of the perfusion change found during treatment for the nine subjects studied with time being plotted on the x-axis 901 and perfusion on the y-axis 902. Analysis shows significant treatment-time interaction ($p = 0.03$) with a significantly ($p<.01$) elevated blood perfusion in the treated arm after 40 minutes of PEMF treatment. The absolute values of baseline perfusion (mv) did not differ between control and treated arms. Analysis of covariance with the baseline perfusion in absolute units (mv) as the covariate also shows an overall difference between treated and control arms ($p<.01$).

A main finding of the present investigational study is that PEMF treatment, when applied in the manner described, is associated with a significant augmentation in their resting forearm skin microvascular perfusion. This augmentation, which averages about 30% as compared with resting pre-treatment levels, occurs after about 40 minutes of treatment whereas no such augmentation is evident in the contralateral non-treated arm. This allows the increased flow of pharmacological, chemical, topical, cosmetic, and genetic agents to the intended tissue target.

Having described embodiments for an apparatus for enhancing pharmacological effects, it is noted that modifications and variations can be made by persons skilled in the art in light of the above teachings.

**Claims**

**1.** An electromagnetic treatment apparatus (200, 600) for enhancing pharmacological effectiveness comprising:

a waveform production means (201, 604) configured to apply a mathematical model that is used to configure waveforms for a given and known target pathway (204, 602), whereby said means (201, 604) produces at least one waveform (500) having at least one selectable waveform parameter (502, 503, 504), and
a coupling device (203, 603) connected to said waveform production means for generating an electromagnetic signal from said at least one waveform, and for applying said electromagnetic signal to a target pathway structure (204, 602) interacting with reactive agents, whereby said applied signal (500) is detectable above thermal noise present at an ion binding location, whereby said target pathway structure (204, 602) is modulated, **characterised in that**
said waveform parameter (502, 503, 504) is selected to maximize at least one of a signal to thermal noise ratio and a Power signal to noise ratio in said target pathway structure (204, 602).

**2.** The electromagnetic treatment apparatus of claim 2, wherein said at least one waveform parameter includes at least one of a frequency component parameter that configures said at least one waveform to repeat between about 0.01 Hz and about 100 MHz according to a mathematical function, a burst amplitude envelope parameter that follows a mathematically defined amplitude function, a burst width parameter that varies at each repetition according to a mathematically defined width function, a peak induced electric field parameter varying between about 1 $\mu$V/cm and about 100 mV/cm in said target pathway structure according to a mathematically defined function, and a peak induced magnetic field parameter varying between about 1 $\mu$T and about 0.1 T in said target pathway structure according to a mathematically defined function.

**3.** The electromagnetic treatment apparatus of claim 2, wherein said defined amplitude function includes at least one of a 1/frequency function, a logarithmic function, a chaotic function, and an exponential function.

**4.** The electromagnetic treatment apparatus of claim 1, wherein said target pathway structure includes at least one of stem cells, molecules, cells, tissues, organs, ions, and ligands.

**5.** The electromagnetic treatment apparatus of claim 1, wherein said reactive agents include at least one of pharmacological agent, chemical agent, topical agent, cosmetic agent, and genetic agent.

**6.** The electromagnetic treatment apparatus of claim 1, wherein said coupling device includes at least one of a reactive coupling device, an inductive coupling device, a capacitive coupling device, and a biochemical coupling device.

**7.** The electromagnetic treatment apparatus of claim 1, wherein the coupling device couples said signal to said target pathway structure to modulate Calcium binding to Calmodulin to enhance said reactive agents' effectiveness.

**8.** The electromagnetic treatment apparatus of claim 1, wherein the coupling device couples said signal to said target pathway- structure to modulate at least one of growth factor production and cytokine production, relevant to enhance said reactive agents' effectiveness.

**9.** The electromagnetic treatment apparatus of claim 8, wherein the growth factor includes at least one of fibroblast growth factors, platelet derived growth factors, interleukin growth factors, and bone morphogenetic protein growth factors.

**10.** The electromagnetic treatment apparatus of claim 1, wherein the coupling device couples said signal to said target pathway structure to modulate angiogenesis and neovascularization to enhance said reactive agents' effectiveness.

**11.** The electromagnetic treatment apparatus of claim 1, wherein the coupling device couples said signal to said target pathway structure to modulate human growth factor production to enhance said reactive agents' effectiveness.

**12.** The electromagnetic treatment apparatus of claim 1, wherein the coupling device couples said signal to said target pathway structure to augment cell and tissue activity to enhance said reactive agents' effectiveness.

**13.** The electromagnetic treatment apparatus of claim 1, wherein the coupling device couples said signal to said target pathway structure to increase cell population to enhance said reactive agents' effectiveness.

**14.** The electromagnetic treatment apparatus of claim 1, wherein the waveform production means, the connecting means, and the coupling device are configured to be lightweight, and portable.

**15.** The electromagnetic treatment apparatus of claim 1, wherein the waveform production means, the connecting means, and the coupling device are incorporated into at least one of a mattress, a mattress pad, a bed, and a positioning device.

**16.** The electromagnetic treatment apparatus of claim 15 wherein the positioning device includes at least one of an anatomical support, an anatomical wrap, and apparel.

**17.** The electromagnetic treatment apparatus of claim 16, wherein said apparel includes at least one of garments, fashion accessories, and footware.

**18.** The electromagnetic treatment apparatus of claim 1, wherein the waveform production means is programmable.

**19.** The electromagnetic treatment apparatus of claim 1, wherein the waveform production means delivers at least one pulsing magnetic signal during a predetermined time.

**20.** The electromagnetic treatment apparatus of claim 1, wherein the waveform production means delivers at least one pulsing magnetic signal during a random time.

**21.** The electromagnetic treatment apparatus of claim 1, further comprising a delivery means for standard physical therapy modalities.

**22.** The electromagnetic treatment apparatus of claim 21, wherein said standard physical therapy modalities includes heat, cold, massage, and exercise.

## Patentansprüche

**1.** Ein Vorrichtung (200, 600) zur elektromagnetischen Behandlung zur Verbesserung der pharmakologischen Wirksamkeit, umfassend:

eine Wellenform-Erzeugungseinrichtung (201, 604) derart konfiguriert, dass ein mathematisches Model angewendet wird, welches zur Konfigurierung von Wellenformen für einen gegebenen und bekannten Ziel-Weg (204, 602) verwendet wird, wobei die Einrichtung (201, 604) mindestens eine Wellenform (500) mit mindestens einem Wellenformparameter (502, 503, 504) erzeugt, und

eine Kupplungsvorrichtung (203, 603), welche mit der Wellenform-Erzeugungseinrichtung (201, 604) zum Erzeugen eines elektromagnetischen Signals von der mindestens einen Wellenform verbundenen ist, zum Applizieren des elektromagnetischen Signal auf die Ziel-Weg-Struktur (204, 602), welche mit reaktiven Agenzien interagiert; wobei das applizierte Signal (500) über dem vorhandenen thermischen Rauschen an einer Ionen-Bindungs-Stelle detektierbar ist, wobei die Ziel-Weg-Struktur (204, 602) moduliert ist, **dadurch gekennzeichnet, dass** der Wellenformparameter (502, 503, 504) ausgewählt ist, um wenigstens eines von ein Verhältnis von Signal zu thermisches Rauschen und ein Verhältnis von Power-Signal zu Rauschen in der Ziel-Weg-Struktur (204, 602) zu maximieren.

**2.** Die Vorrichtung zur elektromagnetischen Behandlung nach Anspruch 1, wobei der mindestens eine Signalparameter wenigstens eines beinhaltet von: ein Frequenzkomponentenparameter, der die mindestens eine Wellenform auf eine Wiederholung zwischen etwa 0.01 Hz und etwa 100 MHz nach einer mathematischen Funktion konfiguriert, ein Burst-Amplitudenhüllkurvenparameter, der einer mathematisch definierten Amplitudenfunktion folgt, einen Burst-Breiteparameter, die bei jeder Wiederholung gemäss einer mathematisch definierten Breite-Funktion variiert, ein spitzenwert-induzierter elektrischer Feldparameter, der zwischen etwa 1 micro V / cm und etwa 100 mV / cm in der Ziel-Weg-Struktur gemäss einer mathematisch definierten Funktion variiert, und ein spitzenwert-induzierter magnetischer Feldparameter, der zwischen etwa 1 micro T und etwa 0,1 T in der Ziel-Weg-Struktur gemäss einer mathematisch definierten Funktion variiert.

**3.** Die Vorrichtung zur elektromagnetischen Behandlung nach Anspruch 2, wobei die definierte Amplitudenfunktion mindestens eines von einer 1/Frequenz-Funktion, einer logarithmischen Funktion, einer chaotischen Funktion und einer Exponentialfunktion beinhaltet.

**4.** Die Vorrichtung zur elektromagnetischen Behandlung nach Anspruch 1, wobei die Ziel-Weg-Struktur mindestens eines von Stammzellen, Moleküle, Zellen, Gewebe, Organe, Ionen und Liganden umfasst.

**5.** Die Vorrichtung zur elektromagnetischen Behandlung nach Anspruch 1, wobei die reaktiven Agenzien mindestens eines von pharmakologischer Wirkstoff, chemisches Agens, topisches Agens, kosmetisches Agens und genetische Agens beinhaltet.

**6.** Die Vorrichtung zur elektromagnetischen Behandlung nach Anspruch 1, wobei die Kupplungsvorrichtung mindestens eines aus eine reaktive Kupplungsvorrichtung, eine induktive Kupplungsvorrichtung, eine kapazitive Kupplungsvorrichtung und eine biochemische Kupplungsvorrichtung beinhaltet.

**7.** Die Vorrichtung zur elektromagnetischen Behandlung nach Anspruch 1, wobei die Kupplungsvorrichtung das Signal zu der Ziel-Weg-Struktur koppelt, um Calcium-Bindung an Calmodulin zur Verbesserung der Wirksamkeit der reaktiven Agenzien zu modulieren.

**8.** Die Vorrichtung zur elektromagnetischen Behandlung nach Anspruch 1, wobei die Kupplungsvorrichtung das Signal zu der Ziel-Weg-Struktur koppelt, um mindestens eines von Produktion von Wachstumsfaktoren und Produktion von Cytokinen zur Verbesserung der Wirksamkeit der reaktiven Agenzien zu modulieren.

**9.** Die Vorrichtung zur elektromagnetischen Behandlung nach Anspruch 8, wobei der Wachstumsfaktor mindestens eines von Fibroblasten-Wachstumsfaktoren, aus Thrombozyten gewonnenen Wachstumsfaktoren, Interleukin-Wachstumsfaktoren, knochenmorphogenetisches- Protein- Wachstumsfaktoren beinhaltet.

**10.** Die Vorrichtung zur elektromagnetischen Behandlung nach Anspruch 1, wobei die Kupplungsvorrichtung das Signal zu der Ziel-Weg-Struktur koppelt, um Angiogenese und Neovaskularisierung zur Verbesserung der Wirksamkeit der reaktiven Agenzien zu modulieren.

**11.** Die Vorrichtung zur elektromagnetischen Behandlung nach Anspruch 1, wobei die Kupplungsvorrichtung das Signal zur der Ziel-Weg-Struktur koppelt, um die Produktion von humanen Wachstumsfaktoren zur Verbesserung der Wirksamkeit der reaktiven Agenzien zu modulieren.

**12.** Die Vorrichtung zur elektromagnetischen Behandlung nach Anspruch 1, wobei die Kupplungsvorrichtung das Signal zur der Ziel-Weg-Struktur koppelt, um Zell und Gewebe-Aktivität zur Verbesserung der Wirksamkeit der reaktiven Agenzien zu erhöhen.

**13.** Die Vorrichtung zur elektromagnetischen Behandlung nach Anspruch 1, wobei die Kupplungsvorrichtung das Signal zur der Ziel-Weg-Struktur koppelt, um die Zellpopulation zur Verbesserung der Wirksamkeit der reaktiven Agenzien zu erhöhen.

**14.** Die Vorrichtung zur elektromagnetischen Behandlung nach Anspruch 1, wobei die Wellenform-Erzeugungseinrichtung, die Verbindungsmittel und die Kupplungseinrichtung so konfiguriert sind, dass sie leicht und tragbar sind.

**15.** Die Vorrichtung zur elektromagnetischen Behandlung nach Anspruch 1, wobei die Wellenform-Erzeugungseinrichtung, die Verbindungsmittel und die Kupplungseinrichtung in mindestens eines von einer Matratze, ein Pad, ein Bett und eine Positioniereinrichtung aufgenommen sind.

**16.** Die Vorrichtung zur elektromagnetischen Behandlung nach Anspruch 15, wobei die Positioniereinrichtung mindestens eines von ein anatomischer Träger, eine anatomische Umhüllung und Bekleidung umfasst.

**17.** Die Vorrichtung zur elektromagnetischen Behandlung nach Anspruch 16, wobei die Bekleidung zumindest eines von Kleidungsstücke, modische Accessoires und Schuhen umfasst.

**18.** Die Vorrichtung zur elektromagnetischen Behandlung nach Anspruch 1, wobei die Wellenform-Erzeugungseinrichtung programmierbar ist.

**19.** Die Vorrichtung zur elektromagnetischen Behandlung nach Anspruch 1, wobei die Wellenform-Erzeugungseinrichtung mindestens ein pulsierendes magnetisches Signal während einer vorbestimmten Zeit liefert.

**20.** Die Vorrichtung zur elektromagnetischen Behandlung nach Anspruch 1, wobei die Wellenform-Erzeugungseinrichtung mindestens ein pulsierendes magnetisches Signal während einer zufälligen Zeit liefert.

**21.** Die Vorrichtung zur elektromagnetischen Behandlung nach Anspruch 1, ferner umfassend ein Zuführmittel für physische Standardtherapie Modalitäten.

**22.** Die Vorrichtung zur elektromagnetischen Behandlung nach Anspruch 1, wobei die physische Standardtherapie Modalitäten Hitze, Kälte, Massage und Bewegung beinhalten.

## Revendications

**1.** Appareil de traitement électromagnétique (200, 600) pour renforcer l'efficacité pharmacologique comprenant :

des moyens de production de forme d'onde (201, 604) configurés pour appliquer un modèle mathématique qui est utilisé pour configurer des formes d'onde pour une trajectoire cible donnée et connue (204, 602), moyennant quoi lesdits moyens (201, 204) produisent au moins une forme d'onde (500) ayant au moins un paramètre de forme d'onde sélectionnable (502, 503, 504), et

un dispositif de couplage (203, 603) connecté auxdits moyens de production de forme d'onde pour générer un signal électromagnétique au moins à partir de ladite forme d'onde, et pour appliquer ledit signal électromagnétique à une structure de trajectoire cible (204, 602) interagissant avec des agents réactifs, moyennant quoi ledit signal appliqué (500) est détectable au-dessus du bruit thermique présent au niveau d'un emplacement de liaison ionique, moyennant quoi ladite structure de trajectoire cible (204, 602) est modulée, **caractérisé en ce que** ledit paramètre de forme d'onde (502, 503, 504) est choisi pour maximiser au moins l'un parmi un rapport signal sur bruit thermique et un rapport signal de puissance sur bruit dans ladite structure de trajectoire cible (204, 602).

**2.** Appareil de traitement électromagnétique selon la revendication 2, dans lequel ledit au moins un paramètre de forme d'onde comprend au moins un paramètre de composante de fréquence qui configure au moins ladite forme d'onde pour répéter entre environ 0,01 Hz et environ 100 MHz selon une fonction mathématique, un paramètre d'enveloppe d'amplitude de salve qui suit une fonction d'amplitude définie mathématiquement, un paramètre de largeur de salve qui varie à chaque répétition selon une fonction de largeur définie mathématiquement, un paramètre de champ électrique induit par un pic variant entre environ 1 $\mu$V/cm et environ 100 mV/cm dans ladite structure de trajectoire cible selon une fonction définie mathématiquement, et un paramètre de champ magnétique induit par un pic variant entre 1 $\mu$T et environ 0,1 T dans ladite structure de trajectoire cible selon une fonction définie mathématiquement.

**3.** Appareil de traitement électromagnétique selon la revendication 2, dans lequel ladite fonction d'amplitude définie comprend au moins l'une parmi une fonction 1/fréquence, une fonction logarithmique, une fonction chaotique et une fonction exponentielle.

**4.** Appareil de traitement électromagnétique selon la revendication 1, dans lequel ladite structure de trajectoire cible comprend au moins une structure parmi les cellules souches, les molécules, les cellules, les tissus, les organes, les ions et les ligands.

**5.** Appareil de traitement électromagnétique selon la revendication 1, dans lequel lesdits agents réactifs comprennent au moins l'un parmi un agent pharmacologique, un agent chimique, un agent topique, un agent cosmétique et un agent génétique.

**6.** Appareil de traitement électromagnétique selon la revendication 1, dans lequel ledit dispositif de couplage comprend au moins l'un parmi un dispositif de couplage réactif, un dispositif de couplage inductif, un dispositif de couplage capacitif et un dispositif de couplage biochimique.

**7.** Appareil de traitement électromagnétique selon la revendication 1, dans lequel le dispositif de couplage couple ledit signal à ladite structure de trajectoire cible pour moduler la liaison du calcium à la calmoduline pour renforcer l'efficacité desdits agents réactifs.

**8.** Appareil de traitement électromagnétique selon la revendication 1, dans lequel le dispositif de couplage couple ledit signal à ladite structure de trajectoire cible pour moduler au moins une production de facteur de croissance et une production de cytokine, aptes à renforcer l'efficacité desdits agents réactifs.

**9.** Appareil de traitement électromagnétique selon la revendication 8, dans lequel le facteur de croissance comprend au moins l'un parmi des facteurs de croissance des fibroblastes, des facteurs de croissance dérivés des plaquettes, des facteurs de croissance des interleukines et des facteurs de croissance des protéines morphogénétiques osseuses.

**10.** Appareil de traitement électromagnétique selon la revendication 1, dans lequel le dispositif de couplage couple ledit signal à ladite structure de trajectoire cible pour moduler l'angiogenèse et la néovascularisation afin de renforcer l'efficacité desdits agents réactifs.

**11.** Appareil de traitement électromagnétique selon la revendication 1, dans lequel le dispositif de couplage couple ledit signal à ladite structure de trajectoire cible pour moduler la production du facteur de croissance humain afin de renforcer l'efficacité desdits agents réactifs.

**12.** Appareil de traitement électromagnétique selon la revendication 1, dans lequel le dispositif de couplage couple ledit signal à ladite structure de trajectoire cible pour augmenter l'activité cellulaire et tissulaire afin de renforcer l'efficacité desdits agents réactifs.

**13.** Appareil de traitement électromagnétique selon la revendication 1, dans lequel le dispositif de couplage couple ledit signal à ladite structure de trajectoire cible pour accroître la population cellulaire afin de renforcer l'efficacité desdits agents réactifs.

**14.** Appareil de traitement électromagnétique selon la revendication 1, dans lequel les moyens de production de forme d'onde, les moyens de connexion et le dispositif de couplage sont configurés de sorte à être légers et portables.

**15.** Appareil de traitement électromagnétique selon la revendication 1, dans lequel les moyens de production de forme d'onde, les moyens de connexion, et le dispositif de couplage sont incorporés dans au moins l'un parmi un matelas, un sur-matelas, un lit ou un dispositif de positionnement.

**16.** Appareil de traitement électromagnétique selon la revendication 15, dans lequel le dispositif de positionnement comprend au moins parmi un support anatomique, une bande anatomique et d'un article d'habillement.

**17.** Appareil de traitement électromagnétique selon la revendication 16, dans lequel ledit article d'habillement comprend au moins l'un parmi les vêtements, les accessoires de mode et les chaussures.

**18.** Appareil de traitement électromagnétique selon la revendication 1, dans lequel les moyens de production de forme d'onde sont programmables.

**19.** Appareil de traitement électromagnétique selon la revendication 1, dans lequel les moyens de production de forme d'onde délivrent au moins un signal magnétique pulsatif pendant une durée prédéterminée.

**20.** Appareil de traitement électromagnétique selon la revendication 1, dans lequel les moyens de production de forme d'onde délivrent au moins un signal magnétique pulsatif pendant une durée aléatoire.

**21.** Appareil de traitement électromagnétique selon la revendication 1, comprenant en outre des moyens de délivrance pour des modalités thérapeutiques physiques standards.

**22.** Appareil de traitement électromagnétique selon la revendication 21, dans lequel lesdites modalités thérapeutiques physiques standards comprennent la chaleur, le froid, le massage et l'exercice physique.

Apply at least one reactive agent to a target pathway structure
STEP 101
Configuring at least one waveform having at least one waveform parameter to be coupled to the target pathway structure
STEP 102
Selecting a value of the at least one waveform parameter to maximize at least one of a signal to noise ratio and a Power signal to noise ratio so that the at least one waveform is detectable in the target pathway structure above background activity in the target pathway structure at which the reactive agent is applied..
STEP 103
Generating an electromagnetic signal from the configured at least one waveform.
STEP 104
Coupling the electromagnetic signal to the target pathway structure.
STEP 105

FIGURE 1

201
203
202
204
200

FIG. 2

300
301
Batteries
302
Switching Power Supply
303
Microcontroller
304
Main Energy Storage
305
Pulse Shaper
306
Pulse Phase Timing Control
307
Voltage Level Conversion
308
Switching HEXFET
309
Output

FIG. 3

400
401
403

FIG. 4A

402

FIG. 4B

500
501
502
503
504

FIG. 5

600
602
603
603
604
605
601

FIG. 6

700
701
702
703

FIGURE 7

PRF = 0.2G, 1 burst/sec
Myosin Phosphorylation (Treated/Sham)
2.0
1.8
1.6
1.4
1.2
1.0
0.8
0
100
200
300
400
500
600
700
800
900
1000
1100
1200
Burst Width (μsec)
802
801

FIG. 8

SKIN PERFUSION CHANGES
TREATED ARM
CONTROL ARM
PERFUSION (% of Basline)
TREATMENT TIME (min)
140
130
120
110
100
90
80
-10
0
10
20
30
40
50
902
901

FIGURE 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004108208 A